# EUROPÄISCHE PATENTSCHRIFT

(11) **EP 0 998 220 B1**
(45) Veröffentlichungstag und Bekanntmachung des Hinweises auf die Patenterteilung: **25.03.2009**
(21) Anmeldenummer: 98943744.7
(22) Anmeldetag: 20.07.1998
(51) Int. Cl.: A61B 6/04

(54) **VORRICHTUNG UND VERFAHREN ZUR FIXIERUNG UND/ODER KOMPRESSION UND/ODER ABFORMUNG VON KÖRPER(-TEILEN)**
DEVICE AND METHOD FOR FIXING, COMPRESSING OR SHAPING (PARTS) OF THE BODY
DISPOSITIF ET PROCEDE POUR LA FIXATION, LA COMPRESSION OU LE MODELAGE (DE PARTIES) DU CORPS

(30) Priorität: 19.07.1997 DE 19731040
(43) Veröffentlichungstag der Anmeldung: 10.05.2000
(60) Teilanmeldung: 05010002.3
(73) Patentinhaber: Elekta AB (publ), 103 93 Stockholm (SE)
(72) Erfinder: Müller, Christian, 86863 Langenneufnach (DE); Vogele, Michael, 86830 Schwabmünchen (DE)
(74) Vertreter: Downing, Michael Philip
(86) Internationale Anmeldenummer: PCT/EP1998/004501
(87) Internationale Veröffentlichungsnummer: WO 1999/003398

(56) Entgegenhaltungen:
- EP-A- 0 728 446
- US-A- 3 212 497
- US-A- 3 783 863
- US-A- 3 851 644
- US-A- 4 934 320

## Beschreibung

Die Erfindung betrifft eine Vorrichtung zur Fixierung von Körper/Körperteilen, insbesondere des menschlichen Körpers bzw. von menschlichen Körperteilen.

In vielen Bereichen der Humanmedizin oder der medizinischen Forschung und Technik ist eine sichere Fixierung des Patienten oder einer Untersuchungsperson erforderlich. Von größter Bedeutung ist dies insbesondere auf dem Gebiet der diagnostischen Anwendung (Radiologie), der Strahlentherapie oder bei operativen/chirurgischen Eingriffen (Neurochirurgie, HNO, usw.) aber auch bei (akuten oder permanenten) prä- oder postoperativen Versorgungen von Wunden/Verletzungen. Durch die Einbeziehung der Computertechnclogie in Diagnose und Therapie sind die Anforderungen an Genauigkeit und Reproduzierbarkeit sowohl bei der Festlegung eines stereotaktischen Rahmensystems im oder am menschlichen Körper, als auch bei der Fixierung des Körpers selbst, gestiegen.

Als Stand der Technik sind folgende Fixationsmethoden bekannt:

### a) Fixation des Körpers mit Klebebändern oder Manschetten:

Der Körper des Patienten liegt auf einer Schaumstoffunterlage. Quer über den Körper gespannte Bänder fixieren den Patienten auf diese Unterlage. Nachteilig sind dabei folgende Punkte:
- Durch starken Zug der Klebebänder kann es zu Eindrücke, Druckstellen, Verschiebungen und/oder Hautschwellungen kommen;
- nach Abnahme der Halteelemente (Klebebänder) ist eine erneute Repositionierung in genau gleicher Lage kaum mehr möglich, was besonders bei stereotaktischen Operationen und in der Strahlentherapie nachteilig ist;
- der Körper ist nicht ausreichend fixierbar; besonders in seitlicher Richtung (nach lateral) ist die Beweglichkeit zu wenig einschränkbar oder definierbar.

### b) Fixation des Körpers durch Verschraubung im Knochen:

Der Körper des Patienten wird an mehreren Stellen über einen Metallrahmen verschraubt. Nachteilig ist dabei folgendes:
- Die Verschraubung am Knochen stellt eine invasive Methode dar und ist somit nur bei bestimmten Indikationen möglich; und gerechtfertigt;
- die psychische Belastung des Patienten ist erheblich;
- die Methode ist nur auf bestimmte Lagerungen des Patienten anwendbar und behindert den Operateur;
- eine Fixation der Weichteile (Muskel, Bänder, Bindegewebe) ist praktisch nicht möglich.

### c) Fixation durch schalungen:

Hierbei wird der Patient auf einer Art "Luftmatratze" gelegt, welche mit Schaumstoffkügelchen gefüllt ist. Durch Absaugen der Luft in dieser Matratze verfestigt sich diese durch Aneinanderlegen der Schaumstoffkügelchen. Dabei wird die Vakuummatratze dem Körper im ersten Schritt angepaßt und im zweiten Schritt abgesaugt. Durch diese Methode erhält man einen Abdruck des Körpers. Nachteilig ist hierbei:
- die üblicherweise verwendeten "Matratzen" garantieren zwar eine Ruhigstellung, aber keine Fixation. Bei unkooperativen oder narkotisierten Patienten ist eine ausreichende Ruhigstellung praktisch nicht möglich;
- oftmals entsteht ein ungenügender Abdruck des Patienten, da sich die Matratze praktisch nicht an allen Körperpartien exakt anlegen läßt;
- durch Faltenbildung oder zu starkes Andrücken entstehen oftmals Druckstellen, welche vor allem bei narkotisierten Patienten zu Druckstellen und Gewebeverletzungen führen können.

Andere Abdruckmaterialien wie Schienen, thermoplastisches Material, oder Kunstoffabdrucke, Gipse, etc. weisen ähnliche Nachteile auf. Zusätzlich sind diese Methoden noch mit erheblichem finanziellen oder zeitlichen Aufwand verbunden und werden deshalb nur für Langzeitanwandungen verwendet.

Weiterhin ist aus der US-A-3,783,863 eine Vorrichtung gemäß den oberbegrifflichen Merkmalen des Anspruches 1 bekannt. Hierbei wird eine Folie.

Der Erfindung liegt deshalb die Aufgabe zugrunde, eine Vorrichtung zur Fixierung des menschlichen Körpers bzw. von Körperteilen zu schaffen, welche die genannten Nachteile vermeidet, einfach in Aufbau und Anwendung und dabei in hohem Maße pationtenschonend ist.

Die Aufgabe wird gelöst mit einer Vorrichtung gemäß den Merkmalen des Anspruches 1. Bevorzugte weiterbildungen sind Gegenstand der abhängigen Ansprüche.

Wie bei vorstehend beschriebene Vorrichtung erfolgt bei der vorgeschlagenen Vorrichtung die Fixierung des menschlichen Körpers im wesentlichen durch Unterdruckkräfte. über einer (flexiblen/starren) Körperauflagefläche mit Absaugöffnungen wird ein deckenartiges Außenelement gelegt, welches in seinem über den Körper (des Patienten) hinausragenden Randbereich zumindest weitgehend luftdicht an der Körperauflagefläche angesaugt ist. Damit entsteht eine geschlossene Unterdruckkammer, so daß der Körper relativ zu der Auflagefläche exakt und unverschiebbar in allen Richtungen fixiert ist.

Der Patient legt sich hierzu in bevorzugter Ausführung einfach auf die Körperauflagefläche und wird mit dem folienartigen Außenelement "zugedeckt". Der Patient oder sein(e) Körperteil(e) sind nun von dieser beutelartigen Struktur umgeben. Durch "Schlupflöcher", können Teile des Körpers (Arme, Beine, Kopf, Rumpf) die Luft- oder Vakuumkammer verlassen und damit unfixiert bleiben, wobei diese Öffnungen mittels luftdichter Ausführung z.B. Gummibündchen oder luftdichten Manschetten abgegrenzt sind. Nun wird die Luft aus dem Raum zwischen Körperauflagefläche und Außenelement abgesaugt und der Patient in der so entstandenen Unterdruckkammer fixiert.

Durch diese Fixierung mittels Unterdruck kann dabei sichergestellt werden, daß insbesondere nach mehrfacher Benutzung der Vorrichtung eine immer feste, exakt gleichbleibende Fixation ermöglicht wird. Erfindungsgemäß kann nun durch einen Sicherheitsknopf bei Bedarf der Patient selbst (z. B. bei Erbrechen, Panik) den Unterdruck unterbrechen und damit den Körper des Patienten sehr rasch und unkompliziert aus der Fixation lösen, da sich dann das Außenelement "schlagartig" von der Auflagefläche abhebt.

Als Mittel zur Erzeugung von Unterdruck sind vakuumpumpen mit einstellbarer Saugdruckhöhe, Schlauchverbindungen und Absperrventile anwendbar. Diese Einrichtungen können in ihrer vakuum-Wirkung auch gesteuert/geregelt werden. Zur Unterstützung der vakuum-wirkung können durch Einbringung von Poren/Kanälen an dem Außenelement bzw. der Körperauflagefläche flache Hohlräume oder Unterdruckkanäle erzeugt werden, welche eine homogene Absaugung garantieren, beispielsweise mittels einer aus Stoßachutzverpackungen bekannten Luftpolsterfolie mit Noppen. Damit kann das vorstehend beschriebene geschlossene System auch als halboffenes System mit dauernder Luftabsaugung, insbes. zur Ermöglichung der Hautatmung bei längeren Operationen betrieben werden. Neben Luft können auch andere Medien zur Erzeugung des Unterdruckes verwendet werden. Eventuelle "Luftflußbarrieren", die durch die Absaugung erzeugt werden oder diese beeinträchtigen, werden somit verhindert. Druckmeßeinrichtungen dienen zudem der Kontrolle eines etwa gleichbleibenden Druck auf die Körperregionen.

Das deckenartige Außenelement, bevorzugt in Art einer an sich bekannten Wärmeschutz-Alufolie, ist als Einwegartikel steril abpackbar, aber auch einfach zu reinigen und sterilisierbar, so daß die Betriebskosten gering bleiben, da alle wesentlichen Teile wiederverwendet werden können.

Besteht das Ziel nicht nur in einer Fixation, sondern in einer reproduzierbaren Lagerung, so können zusätzlich vorgefertigte Schalungen, Bänder, Vakuummatratzen verwendet werden, welche die Reproduzierbarkeit erhöhen. Da durch das Absaugen der Körper (des Patienten), insbesondere bei konkaven Auflageflächen in die jeweils gleiche Position "wandert", kann eine hohe Repositioniergenauigkeit geschaffen werden. Dabei kann durch Gleitfolien der Widerstand zwischen Patient und Schalung bzw. zwischen Patient und Außenelement oder Körperauflagefläche besonders gering gehalten werden, so daß das Einnehmen der richtigen Position ohne hohen Kraftaufwand durch Scherkräfte erfolg.

Die beschriebene Fixation kann entweder für den ganzen Körper angewendet werden oder auch nur für einzelne Körperteile. So können z.B. Knochenbrüche und komplizierte Frakturen anstatt mit Gipsverbänden mittels beschriebener Vakuumfixation ruhiggestellt werden. Bei einer bevorzugten Methode werden Schalungen oder Schienungen mit der gewünschten, zu erzielenden Anatomie mit in den Vakuumverband eingearbeitet. Das angelegte Vakuum bewirkt, daß eine homogene Anlage der Schalungen an den Körperteilen erzielt wird bzw. sich die Körperteile diesen Strukturen anlegen. Bei offenen wunden oder großen Schnittverletzungen, die starke Narbenbildung zur Folge haben, bewirkt der Vakuumverband, daß sich die Wundränder aneinanderlegen und somit glattflächig schließen lassen. So kann in manchen Fällen auf eine Hautnaht an der Hautoberfläche verzichtet werden.

Im folgenden werden bevorzugte Ausführungsbeispiele anhand der Zeichnungen näher beschrieben und erläutert, Hierbei zeigen:

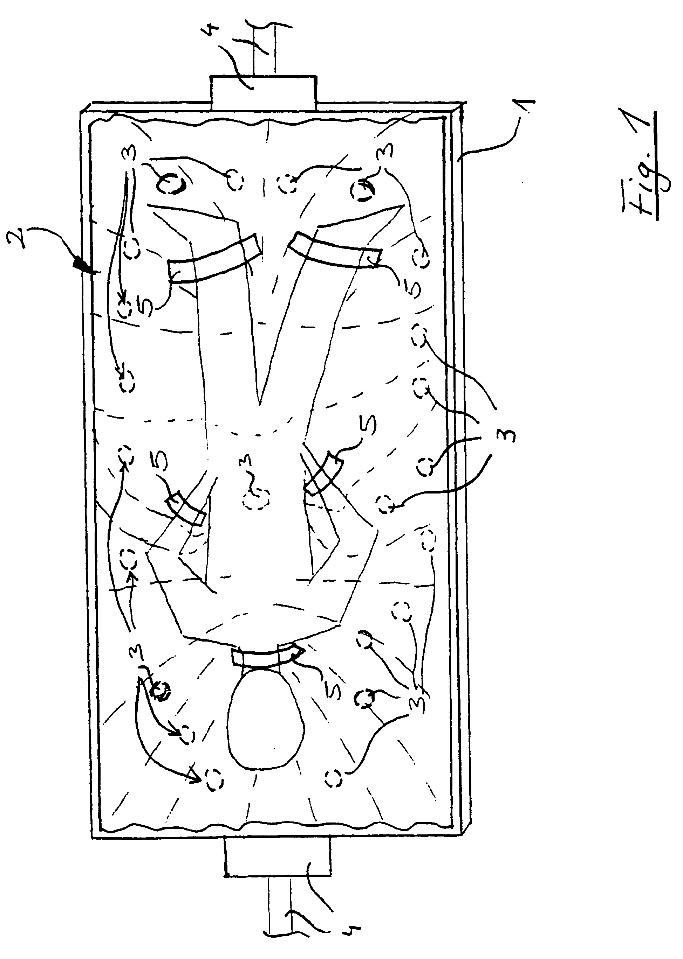

- Fig. 1: eine Vorrichtung zur Fixierung des Körpers eines Patienten in schematischer Draufsicht;

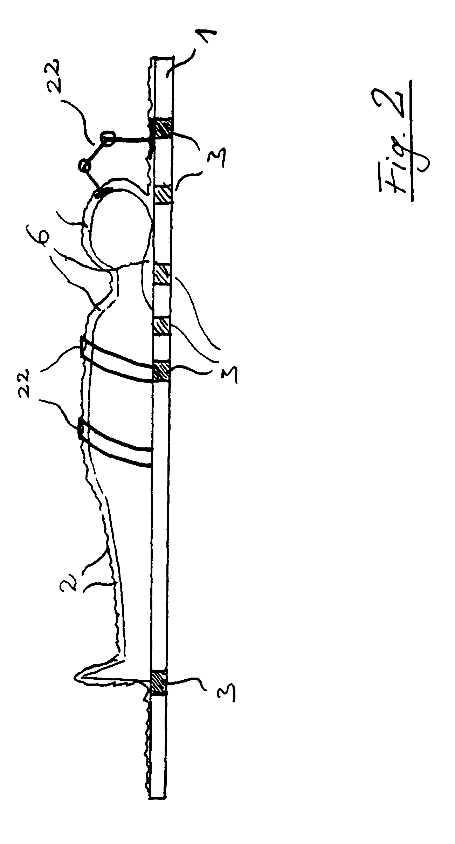

- Fig. 2: die Vorrichtung gemäß Fig. 1 in seitenansicht;
- Fig. 3: die Vorrichtung gemäß Fig. 1 und 2 im Querschnitt.

Ein bevorzugtes Ausführungsbeispiel weist eine aus leichtem und röntgendurchlässigem Material gefertigte Körperauflagefläche 1 auf, an die in Modulbauweise (Erweiterbarkeit, Reduzierbarkeit) weitere Plattenteile angeschlossen oder abgenommen werden können. Als derartige Platten können beispielsweise extrudierte Doppelsteg-Kunststoffplatten aus PMMA verwendet werden, wie sie z.B aus Dacheindeckungen bekannt sind. Diese Hohlkammerplatten bilden zugleich in ihrem Innern eine Absaugkammer aus, die mit einer vielzahl von Ansaugöffnungen 3 in Verbindung steht. Die Körperauflagefläche 1 ist somit leicht und transportabel. Sie kann an ihren Seiten auch mit stabilen Aufhängevorrichtungen 4, beispielsweise Verschraubungen, mit einem Operationstisch verbunden sein. Die Körperauflagefläche 1 kann an diesen Anschlüssen gegenüber dem OP-Tisch in horizontaler und in vertikaler Richtung verstellt und gedreht werden, wobei in jeder Lage ein hohes Maß an Festigkeit gewährleistet ist.

Auf der als Bezugsbasis dienenden Körperauflagefläche 1 ist ein folienartiges Außenelement 2 aufgelegt. Zur Befestigung des Außenelements 2 am umlaufenden Randbereich dienen insbesondere luftdichte Reißverschlüsse, Klett-, Klemm- und Steckverbindungen 9, welche sich einfach und schnell bedienen lassen und einen festen und dichten Abschluß zur Außenluft ermöglichen. Es sei darauf hingewiesen, daß bei geeigneter Materialwahl, insbesondere einer dünnen Kunststoff- oder Metallfolie als Außenelement 2 auch auf eine solche Befestigung am Randbereich verzichtet werden kann, da sich ein solches Außenelement 2 selbst an dem Randbereich der Körperauflagefläche 1 glattflächig anschmiegt und damit abdichtet, insbesondere bei einer Innengummierung oder ähnlichen Beschichtung. Das Außenelement 2 kann jedoch als Kunststoffseil ähnlich einem Deckel oder einer konvexen Schale ebenso wie die (konkave) Körperauflagefläche 1 relativ formstabil ausgebildet sein, die sich dann erst unter Vakuumanlegung an den Körper des Patienten anlegen.

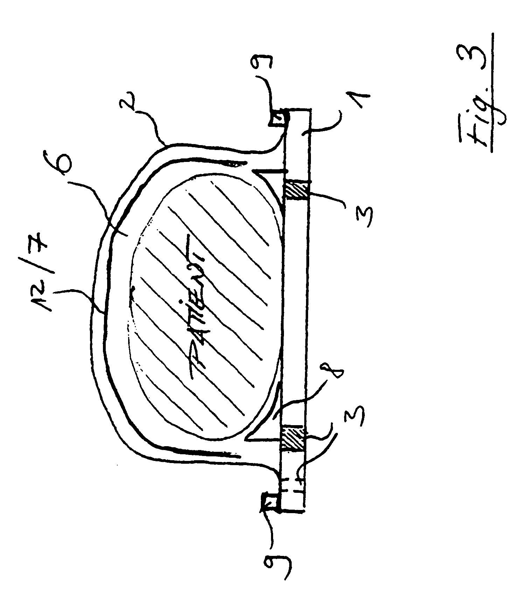

Das Außenelement 2 wird zunächst in Art einer Bettdecke oder eines Tuches locker auf den Patienten gelegt und am Außenrandbereich der Körperauflagefläche angedrückt bzw. befestigt. Daran anschließend wird über Ansaugöffnungen 3 das Medium (insbesondere Luft) in der so gebildeten Unterdruckkammer 6 (vgl. Fig. 3) abgesaugt. Durch die Absaugung legt sich die als Außenelement 2 bevorzugterweise verwendete Folie auf dem Patienten zunehmend an. Über Meßsonden kann dabei der Unterdruck in der Unterdruckkammer 6 permanent kontrolliert werden.

In das Außenelement 2 eingesetzte Durchgriffsöffnungen 5 mit Manschetten ermöglichen es, daß Körperpartien die Unterdruckkammer 6 verlassen können und damit einer Fixation entzogen sind. Somit können auch die Atemwege und Ohren (Trommelfellinflation) vom angelegten Vakuum freigehalten sein.

Die Unterdruckkammer 6 kann in einfachster Ausführung ein gegenüber der Umgebung abgeschlossenes System bilden. Einmaliges Absaugen und verschließen des Systems, beispielsweise mittels Absperrventilen läßt den in der Unterdruckkammer 6 entstanden Unterdruck lange konstant aufrechterhalten. Eine weitere Möglichkeit, insbesondere bei längeren Operationen ist jedoch, durch die Einbringung von Poren bzw. Luftkanälen 23 das System "halboffen" zu gestalten. Der erwünschte Unterdruck in der Unterdruckkammer 6 kann dann mit einer permanenten Absaugung (unter "Flow") aufrechterhalten werden. Ebenso ist damit die Einbringung von geeigneten Medien (Kühlflüssigkeit, Medikamente, Nährlösungen, usw.) damit möglich. Auch können vom Körper abgegebene Stoffe (Blut, Wundsekrete, ...) damit in vorteilhafter Weise abgesaugt werden. Eine weitere Anwendung wäre die Absaugung von Fettpölsterchen unter der Haut mit postoperativem Aufrechterhalten des vakuums als Druckverband.

Wie insbesondere in Fig. 3 dargestellt, liegt der Patient P dabei bevorzugt auf einem Auflageelement 8, welches sich zwischen Patient und Körperauflagefläche 1 befindet. Diese luftdurchlässige Patientenauflage 8 ist relativ weich ausgebildet (z.B. Stoff, Fell, Polster, ...) und dient damit zur Erhöhung des Liegekomforts, kann aber auch eine feste, strukturgebende Form aufweisen (z.B. eine Rinne, konkave Kissen usw.) und dadurch eine reproduzierbare Lagerung bei mehrmaligen Eingriffen möglich machen. Ähnliche Einlageelemente 7, analog den Auflageelementen 8 können auch zwischen Außenelement 2 und dem Patient angebracht sein. Die Einlage- und Auflageelenente 7 und 8 können frei verschiebbar und damit dem Körper des Patienten anpaßbar sein, oder auf der Körperauflagefläche 1 befestigt sein. Sie erhöhten die Repostioniergenauigkeit und vergrößern die Anlagefläche auf der Körperauflagefläche 1.

Die Vorteile lassen sich wie folgt zusammenfassen:
a) für die Fixation:
   - homogene Kompression des Körpers (falls erwünscht);
   - maximale Sicht- und Bewegungsfreiheit für den operateur;
   - Positionierung des Körpers in variablen Lagen bei Eingriffen;
   - keine Hautverschiebung und Lageverschiebungen möglich;
   - Anbringungsmöglichkeit von Instrumenten (z.B. Endoskop);
b) allgemeine Vorteile:
   - höchste Stabilität bei geringem Gewicht;
   - Tauglichkeit für diagnostische (CT, MRI) Verfahren;
   - gleichmäßiger Druck an allen Stellen oder erhöhter Druck an Stellen mit eingebrachten Schienelementen;
   - stufenlose Druckregulierung möglich;
   - platzsparender Aufbau;
   - einfache und zeitsparende Handhabung durch die Unterdruckanlegung und daher besonders patientenschonend und hygienisch;
   - hohe Patientensicherheit durch Kontrolle des Unterdruckes in den Unterdruckkammern und der Druckbelastung des Patienten; zudem sofortige Befreiungsmöglichkeit und non-invasive Fixierung.

## Patentansprüche

1. Vorrichtung zur Fixierung von menschlichen Körperteilen oder des menschlichen Körpers, aufweisend:
- eine Körparauflagefläche (1),
- mindestens ein flexibles Außenelement (2), welches frei auf der Körperauflagefläche (1) positionierbar ist und mit der Körperauflagefläche (1) eine den Körper oder das Körperteil umschließende Unterdruckkammer (6) bildet, und
- mindestens eine Absaugöffnung (3), welche mit der Unterdruckkammer (6) in Verbindung steht,
**dadurch gekennzeichnet, dass**
ein Patientenbefreiungsmechanismus mit plötzlichem Abbau des Unterdrucks in der Unterdruckkammer (6) vorgesehen ist, wobei der Unterdruck durch den Patienten selbst mittels eines Sicherheitsknopfes unterbrechbar ist.

2. Vorrichtung nach Anspruch 1,
**dadurch gekennzeichnet, daß**
die Körperauflagefläche (1) an einem Eingriffs- oder operationstisch anbringbar ist, insbesondere mittels einer Schwenkaufhängung (4).

3. Vorrichtung nach Anspruche 1 oder 2,
**dadurch gekennzeichnet, daß**
die Absaugöffnungen (3) als einzelne Absaugkanäle ausgebildet sind.

4. Vorrichtung nach einem der Ansprüche 1 bis 3,
**dadurch gekennzeichnet, daß**
die Körperauflagefläche (1) und/oder das Außenelement (2) aus einzelnen Modulen bestehen und aneinanderfügbar sind.

5. Vorrichtung nach einem der Ansprüche 1 bis 4,
**dadurch gekennzeichnet, daß**
vorgeformte oder individuell geformte Patientenauflagen (8) an der Körperauflagefläche (1) und/oder dem Außenelement (2) anlegbar sind.

6. Vorrichtung nach Anspruch 5,
**dadurch gekennzeichnet, daß**
als Patientenauflage (8) Elemente zur Verringerung des Reibungewiderstands, insbesondere Gleitfolien, oder zur Erhöhung des Reibungswiderstands, insbesondere eine Gummimatte, an dem Außenelement (2) und/oder der Körperauflagefläche (1) anbringbar sind.

7. Vorrichtung nach einem der Ansprüche 1 bis 6,
**dadurch gekennzeichnet, daß**
auf der Körperauflagefläche (1) Markierungen und/oder steckbohrungen zur Repositionierung eines Patienten (P) angebracht sind.

8. Vorrichtung nach einem der Ansprüche 1 bis 7,
**dadurch gekennzeichnet, daß**
Fixationsvorrichtungen (22) für den Körper oder für Körperteile an die Körperauflagefläche (1) anschließbar sind.

9. Vorrichtung nach einem der Ansprüche 1 bis 8,
**dadurch gekennzeichnet, daß**
Druckmesseinrichtungen zur Kontrolle der (Unter-) Druckverteilung in der Unterdruckkammer (6) vorgesehen sind.

10. Vorrichtung nach einem der Ansprüche 1 bis 9,
**dadurch gekennzeichnet, daß**
das Außenelement (2) und/oder die Korperauflagefläche (1) an wenigstens einer Stelle eine Durchgrifföffnung (5) aufweist, die ringsum abgedichtet sind.

## Claims

1. Apparatus for fixing human body parts or the human body, comprising:
- a body support surface (1),
- at least one flexible outer element (2) which can be positioned freely on the body support surface (1) and forms with the body support surface (1) a vacuum chamber (6) surrounding the body or the body part, and
- at least one suction opening (3) which communicates with the vacuum chamber (6),
**characterized in that**
a patient release mechanism with abrupt removal of the vacuum in the vacuum chamber (6) is provided, wherein the vacuum can be interrupted by the patient himself by means of a security button.

2. Apparatus according to claim 1, **characterized in that** the body support surface (1) can be fitted to a procedure or operating table, especially by means of a pivot suspension (4).

3. Apparatus according to claim 1 or 2, **characterized in that** the suction openings (3) are formed as individual suction channels.

4. Apparatus according to any of claims 1 to 3, **characterized in that** the body support surface (1) and/or the outer element (2) consist of individual modules and can be joined together.

5. Apparatus according to any of claims 1 to 4, **characterized in that** preformed or individually formed patient supports (8) can be applied to the body support surface (1) and/or the outer element (2).

6. Apparatus according to claim 5, **characterized in that** elements for reducing the frictional resistance can be applied as patient supports (8), especially slip films, or for increasing the frictional resistance, especially a rubber mat, to the outer element (2) and/or the body support surface (1).

7. Apparatus according to any of claims 1 to 6, **characterized in that** markings and/or peg bores are provided on the body support surface (1) for repositioning a patient (P).

8. Apparatus according to any of claims 1 to 7, **characterized in that** fixation devices (22) for the body or for body parts can be attached to the body support surface (1).

9. Apparatus according to any of claims 1 to 8, **characterized in that** pressure measuring devices are provided for controlling the (vacuum) pressure distribution in the vacuum chamber (6).

10. Apparatus according to any of claims 1 to 9, **characterized in that** the outer element (2) and/or the body support surface (1) comprises an access opening (5) at at least one point, which is sealed off around itself.

## Revendications

1. Dispositif pour la fixation de parties du corps humain ou du corps humain, présentant
- une surface d'appui du corps (1),
- au moins un élément extérieur flexible (2) qui peut être positionné librement sur la surface d'appui du corps (1) et qui forme avec la surface d'appui du corps (1) une chambre à dépression (6) entourant le corps ou la partie du corps, et
- au moins une ouverture d'aspiration (3) qui communique avec la chambre à dépression (6),
**caractérisé en ce qu'**il est prévu un mécanisme de libération du patient avec suppression soudaine de la dépression dans la chambre à dépression (6), la dépression pouvant être interrompue par le patient lui-même au moyen d'un bouton de sécurité.

2. Dispositif selon la revendication 1, **caractérisé en ce que** la surface d'appui du corps (1) peut être posée sur une table d'intervention ou d'opération, en particulier au moyen d'une suspension pivotante (4).

3. Dispositif selon la revendication 1 ou 2, **caractérisé en ce que** les ouvertures d'aspiration (3) sont formées de canaux d'aspiration séparés.

4. Dispositif selon l'une des revendications 1 à 3, **caractérisé en ce que** la surface d'appui du corps (1) et/ou l'élément extérieur (2) sont composés de différents modules et peuvent être assemblés l'un à l'autre.

5. Dispositif selon l'une des revendications 1 à 4, **caractérisé en ce que** des appuis du patient (8) préformés ou formés individuellement peuvent être posés sur la surface d'appui du corps (1) et/ou l'élément extérieur (2).

6. Dispositif selon la revendication 5, **caractérisé en ce que** comme appuis du patient (8), des éléments destinés à réduire la résistance au frottement, en particulier des films de glissement, ou à augmenter la résistance au frottement, en particulier un matelas en caoutchouc, peuvent être placés sur l'élément extérieur (2) et/ou la surface d'appui du corps (1).

7. Dispositif selon l'une des revendications 1 à 6, **caractérisé en ce que** des marquages et/ou des trous d'insertion pour le repositionnement d'un patient (P) sont disposés sur la surface d'appui du corps (1).

8. Dispositif selon l'une des revendications 1 à 7, **caractérisé en ce que** des dispositifs de fixation (22) pour le corps ou pour des parties du corps peuvent être raccordés à la surface d'appui du corps (1).

9. Dispositif selon l'une des revendications 1 à 8, **caractérisé en ce que** des dispositifs de mesure de la pression sont prévus pour contrôler la répartition de la (basse) pression dans la chambre à dépression (6).

10. Dispositif selon l'une des revendications 1 à 9, **caractérisé en ce que** l'élément extérieur (2) et/ou la surface d'appui du corps (1) présentent à au moins un endroit une ouverture de passage des mains (5) qui est fermée hermétiquement sur son pourtour.
